Europäisches Patentamt

(19) **European Patent Office**

Office européen des brevets

(11) Publication number: **0 014 022**
**B1**

(12) **EUROPEAN PATENT SPECIFICATION**

(45) Date of publication of patent specification: **14.11.84**

(51) Int. Cl.³: **A 43 D 1/02, A 61 B 5/10**

(21) Application number: **80200076.0**

(22) Date of filing: **25.01.80**

(54) **Foot-size measuring apparatus.**

(30) Priority: **25.01.79 NL 7900596**

(43) Date of publication of application:
**06.08.80 Bulletin 80/16**

(45) Publication of the grant of the patent:
**14.11.84 Bulletin 84/46**

(84) Designated Contracting States:
**BE DE FR GB IT**

(56) References cited:
**FR-A-2 188 819**
**FR-A-2 258 141**
**US-A-3 457 647**
**US-A-4 086 580**

(73) Proprietor: **Technisch Advies- en Handelsbureau Hoogstraat C.V.**
**Energiestraat 9**
**NL-8263 AG Kampen (NL)**

(72) Inventor: **Brinkgreve, Peter**
**Willibrorduslaan 61b**
**5581 GB Waalre (NL)**
Inventor: **van Dijk, Dominique Maria**
**Willibrorduslaan 80**
**5581 GB Waalre (NL)**
Inventor: **Snijders, Christiaan Johannes, Jhr.**
**Hugo v. Berckellaan 4 Postbus 116**
**5670 AC Nuenen (NL)**

(74) Representative: **Morel, Christiaan Frederik**
**MORELPATENT Laarstraat 3**
**NL-8166 GR Emst (Epe) (NL)**

Courier Press, Leamington Spa, England.

## Description

The invention relates to an apparatus for measuring the width and length of a foot, comprising:

(i) a platform adapted to receive the foot placed thereon, with foot aligning means,
(ii) a frame with a foundation plate,
(iii) first and second light sources, level with the foot to be measured, in respectively one end wall and one side wall of the frame,
(iv) first and second movable carriers in respectively the other end wall and the other side wall of the frame, supporting respectively first and second light sensing means, and
(v) an electrical circuit and a read-out panel,

in use the first and second carriers being simultaneously moved parallel to respectively the width and length dimensions of the foot from respective positions in which the light from the respective light sources is obscured by the foot to respective second positions in which the light is incident on the respective light sensing means, the resultant signals from the respective light sensing means being used by the electrical circuit to determine respectively the width and length of the foot and to display them on the read out panel. Such an apparatus for measuring the foot-size is known from the United States Patent 3 357 647.

The foot-size measuring apparatus described in the above mentioned United States Patent consists of a frame work with a foot-receiving platform, being the platform on which the client stands. The foot to be measured can be placed for example with its inner side and heel against two vertical walls, which are perpendicular to each other. However, the result of the measurements depends on the extent that the foot is pressed against the two walls, which are perpendicular to each other. The pressure and the deformation resistance of the tissue of a person's foot differs from person to person meaning that the starting conditions will not always be the same.

Another foot-size measuring apparatus is known from the British Patent 1 489 181. The foot-size measuring apparatus described in this British Patent consists of a frame work which has a transparent foot-receiving platform, which is the platform on which the client stands. The foot to be measured can be placed, for example, with its inner side and heel, against two vertical walls, which are perpendicular to each other. Light is radiated downwards to the foot-receiving platform by means of a light source fixed to a column placed on the frame work. Underneath the platform, are mounted, perpendicular to each other, two banks of photocells, which detect the light radiated through the platform except for the part shadowed by the foot. Beginning at the known maximum length and width of the platform and counting down by means of the photo-cells, the maximum width and length of the foot is reached.

By means of the foot-size measuring apparatus known from the British Patent the shadow of a person's foot is measured, the light source is a single bulb, radiating downwards, so that it can occur that the light-rays will not always be vertical but slanted when they reach the platform so that the shadow of the foot will not be correct. Also the result of the measurements depends on the extent that the foot is pressed against the two walls, which are perpendicular to each other. The pressure and the deformation resistance of the tissue of a person's foot differs from person to person meaning that the starting conditions will not always be the same. Another considerable problem is the fact, that to obtain the correct dimensions of the person's foot, the foot has to be placed under the total load, that is to say with the person's full weight on the platform. The frame of this measuring apparatus has to be as rigid as possible, so that no sagging will occur especially because the accurate measuring device is placed underneath the foot-receiving platform.

That it is possible to determine the dimension of an object without touching it, is, for example, known from the French Patent 2 188 819. In this French Patent is described a method, by which a laser beam, mirrors and photo-cells are used to determine the dimensions of a foot, for example a rotor for a turbine.

The aim of the invention is to provide a foot-size measuring-apparatus of a simple, solid and very light construction, with which can be carried out accurate measurements to determine the maximum width and length of a foot, while there is no physical contact at all. This is achieved according to the invention with a foot-size measuring apparatus of the mentioned kind in that,

(vi) the first and second light sensing means are respectively a first and second vertical bank of respectively first and second light sensors,
(vii) in use the first light sensing means moves from a first position where light is incident on all the first sensors, through a second position aligned with one of the two side extremities of the foot in which the light to at least one of the first sensors is obscured by the foot, to a third position at the other side extremity of the foot in which the light reaches all the first sensors again,
(viii) in use the second light sensing means moves from a fourth position where light is incident on all the second sensors, through a fifth position aligned with one of the two end extremities of the foot in which the light to at least one of the second sensors is obscured by the foot, to a sixth position at the other end extremity of the foot in which the light reaches all the second sensors again, and the electrical circuit being adapted to produce and process:

(ix) the signals produced at the second and third positions to determine the width of the foot, and

(x) the signals produced at the fifth and sixth positions to determine the length of the foot. So without having any physical contact with the foot, the maximum length and width of the foot is determined.

According to the invention said measuring apparatus can comprise a removable foot-receiving platform which can be placed inside the frame of the apparatus directly on the floor. So the forces of the load, work directly on the platform and there will be no forces working on the measuring and driving arrangement of the measuring apparatus, so it can be of a light construction. Holes can be made in the foot-receiving platform so it can be taken out and cleaned easily. For positioning the foot an axial-cross can be provided on the platform surface.

The above and other objects, advantages and features of the invention will be best understood from the following description taken in conjunction with the accompanying drawings, in which:

Figure 1 is a top-view of a foot;

Figure 2a is a side-view of a measuring apparatus according to the invention for detecting the dimensions of a foot without any physical contact with the foot;

Figure 2b and 2c are side-views of a foot together with some important dimensions;

Figure 3 is a view of the side-walls, the foot-receiving platform and the foundation plate of the measuring apparatus according to the invention;

Figure 4a—4b are respectively a side-view and a top-view of the oblong lamps used in the apparatus;

Figure 5 is a view of a slitted disc with a driving mechanism and a photo-detecting system;

Figure 6 is a view of a straight slitted strip which can be used with the measuring apparatus;

Figure 7 is as example a part of a slitted strip with some slits;

Figure 8 is a system diagram of a measuring arrangement;

Figure 9 is a top-view of the foot-receiving platform and the foundation plate together with several elements;

Figure 10 is a side-view of a carrier placed on guides together with a container for the light sensors.

For measuring the dimension of a foot always three elements are involved: the object to be measured i.e. the foot; the measuring apparatus together with a scale; and the person who carries out the measurement and usually also interprets the results, for example a member of the personnel of the shop.

For positioning the foot in the measuring apparatus in fact two directions have to be determined. As the measurements in the lateral, direction are carried out perpendicular to the measurement in the longitudinal direction, it is sufficient to position the foot just in one direction and preferably in the longitudinal direction as the length of the foot is much larger than the width.

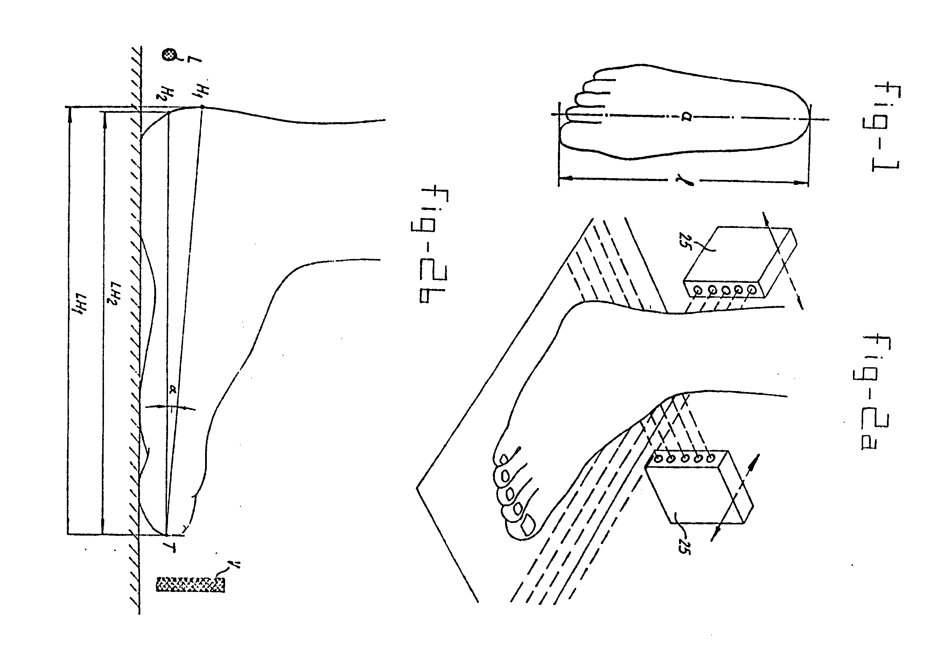

In figure 1 line a, the so called sagittal line which cuts the heel and second toe in two, is the exact longitudinal direction of the foot. The exact longitudinal dimension of the foot is obtained if the measurements are carried out parallel to this direction and displacement of the foot along this line a has no influence on the measurement of the width of the foot. As in fact always stockings are worn there will be differences between the different measurements. For that reason the measurements are carried out preferably with those stockings worn normally in the new shoes. Of course during the measurements of a foot reality will be aimed for as much as possible, so in conformity to reality the foot has to be under its normal weight. A measurement can be done under half-load i.e. the person's weight is divided between both feet, or under full load, i.e. the person's full weight is on one foot. The condition under which the measurements are carried out depends on the different circumstances. For the reading of the read-out panel, a digital or analogue reading in millimeters is used as this unit is the internationally accepted standard Mondopoint foot-size unit according to the ISO standard 2816. The reading according to the invention will be digital with an accuracy deviation in the longitudinal dimension of about 0.5 mm and in the lateral dimension of about 0.2 mm. It is important to measure with such an accuracy and a high reproducibility due to the fact that shoes are fabricated in several different shoe-size indicating systems and that especially in the new Mondo-point system the different classes, i.e. the pitch or steps with which each shoe-size succeeds the next, are smaller than in the previously known systems. The longitudinal size will have steps of 5 mm and the lateral size steps of 3 mm. The smaller the tolerance the easier a foot can be classified in a foot-size system.

The method to measure the size of a foot, with no physical contact, falls into two parts: the release of an initial- and end- or stopsignal of the measurement and the final measurement by means of the circuit.

Figure 2a shows how the photosensing-lightbeam method is carried out, the vertical placed beams of light radiate from one longitudinal side-wall and one lateral side-wall towards each opposite wall and the received by a certain number of vertically placed radiant energy sensing elements mounted in containers 25. Figure 2b shows that if measurements are carried out by a single light-ray, that depending on the altitude of the light-ray with which the measurements are carried out, different results of the length of the foot will be obtained. The same for the measurement of the width of the foot. In general the extreme outside points of the heel, the toe and the inside and outside of the ball of the foot do not lie in the same plane. The consequence is that a light ray at a certain constant altitude will not touch all the most extreme points of the foot; so not the exact length and width of the foot will be determined.

Figure 2b shows the exact length $LH_1$ of the foot being the projection of the line $H_1T$ on the sole-plate. This line $H_1T$ makes an angle $\alpha$ to the sole-plate. If a ray of light L were to move at a certain altitude along the foot, the smaller dimension $LH_2$ would be determined. In principle the ray of light has to be moved along the foot at an angle $\alpha$. The difficulty is that the angle $\alpha$ differs for each individual foot.

Figure 2c shows a contour of a foot with a respective curved line between the points A, B and C, D of which the curvature differs for each foot. However at the extremities of the heel and toe the curved lines approximate to a straight line. So there is no need to speak of the extremities as being one single point, but it is reasonable to speak of straight lines A—B and C—D on which lay the extreme points. So by moving along the foot a narrow vertical rectangular beam of light V instead of a ray of light L, automatically all the extreme points are "covered". Measurements have shown that in principle the use of a narrow beam of light with a height of about 20 mm is sufficient to cover all the extremities. In the measuring apparatus according to the invention the height of the beam in both the longitudinal and lateral directions is preferably 25 mm, although in the lateral direction about 10 mm is sufficient.

There also has to be found a solution for the problem of the weight or load on the person's foot, as basically it is important that the construction of the apparatus should be simple, light and small. This force, caused by the person's weight, can be as high as 150 kgf. The consequence is that the frame of the measuring apparatus has to be so rigid that no deflection will occur. The solution of this problem is found in that the load forces work directly on the platform. This is realised in a construction in which the foot-receiving platform is independent of the framework; the framework together with the foundation plate is constructed in such a way that it surrounds the foot-receiving platform and the measuring device and driving mechanism are built in the inside of the framework.

Figure 3 shows a view of the foot-receiving platform 2 which can be surrounded by the framework and a cover 30 which covers the measuring device and driving mechanism. The dimensions of the platform are, for example, 375x150 mm, so that a foot up to size 55 can be measured. The platform placed directly on the floor can be made out of wood or plastic about 20 mm thick. On the surface of the platform an axial-cross can be provided for easy positioning of the foot.

The foundation plate of the framework of the measuring apparatus serves as a footing for both the measuring device and driving mechanism. The dimension are for example 650x400 mm. The foundation-plate can be made of 10 mm thick multiplex or plastic, because no forces will work on it. The cover is made out of about 8 mm thick plastic and made by vacuum-moulding.

Both for the longitudinal direction and for the lateral direction oblong light sources are used. The holders with the radiant energy sensing means, to determine the interruption of the beam of light by the foot, move along the opposite wall. Preferably half-mirror lamps or half-reflecting lamps 5, having a mirror part 31, are used as light sources.

By using oblong lamps there is no need for seperate carriers for the displacement of small lamps, which otherwise have to be moved synchronously with the radiant energy sensors both in the longitudinal direction and in the lateral direction. So there is less wear and tear and the lamps need not be aligned. Also because of the permanently broad vertical surface of light that is radiated there is no need to synchronize the carriers with the radiant energy sensors. Then, there is no need for an expensive driving mechanism as the driving can be done by means of a steel wire or nylon cord or by means of a lead screw together with a ball-and-socket joint. Also a relatively small motor can be used.

Another method to obtain a luminous surface is by placing a luminous point source for example a halogen lamp in a corner of the two guides of the guide-mechanism. The beam of light radiated along the longitudinal and lateral side-walls is reflected by a mirror placed at an angle of 45 degrees to the direction of movement of the carrier, then towards an oblong plane mirror at the opposite wall of the platform. This oblong plane mirror returns the light in such a way that light falls on the radiant energy sensing means mounted on the carrier at the opposite side-wall.

As light sensing means can be used photocells. A variable resistance can be used to regulate the moment of excitement of the photo-cells so that a possible disadvantage of the incidence of light from the sides can be avoided. Also the measuring apparatus can be calibrated with the rheostat.

If oblong lamps are used as source of light then for the lateral dimension it is sufficient to use only one lamp of a mirror type of a length of about 155 mm, which can be bought in a shop. However for the longitudinal direction there is need of light over a length of 375 mm, so a lamp has to be used which has a greater diameter with all the possible disadvantages thereof. In fact light is needed only to obtain an initial- and stopsignal for the measurements. That is to say that there is no need for light along the reach of the foot. So it is possible to make use of two lamps of about 155 mm, which are placed in a line in such a way that the connecting point is always in the shadow of the foot.

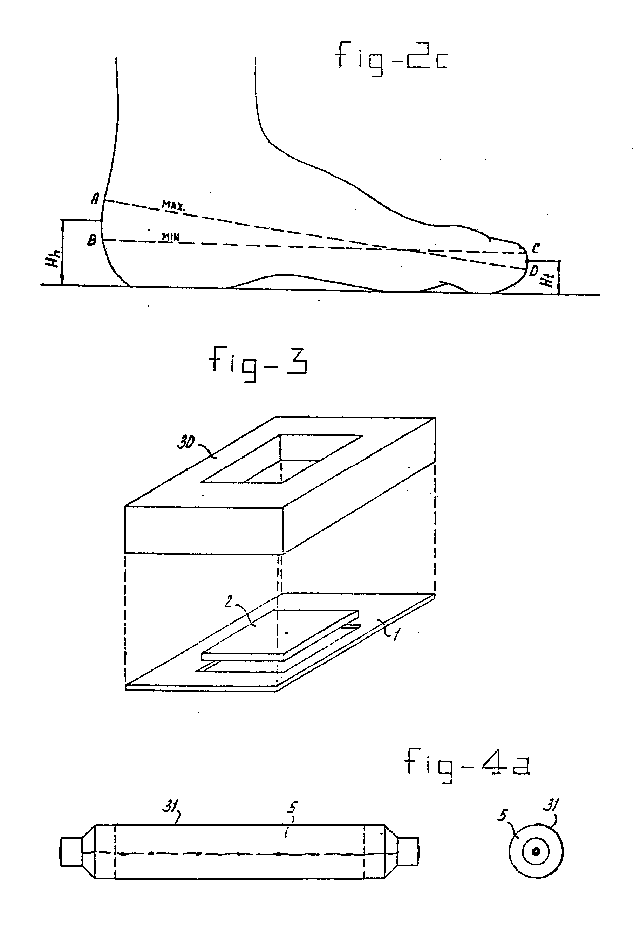

Figure 4b shows the illuminated and not-illuminated parts 32 and 33. So for the measuring apparatus three identical lamps 5 of 25 watt can be used.

The measurement of the dimensions of the foot is in fact the measurement of the time during which, the light falling on both carriers with the photo-sensors is interrupted by the foot. To measure this period of time it is possible to use a slitted disk 17a which for example is coupled to the carriers. In the rim of each disk a certain number of slits are milled and on one side of the rim is mounted a source of light and opposite to it on the other side a photo-sensor or photocell. Each change from light to darkness and from darkness to light, which is generated by turning the disk in the beam of light, is converted into pulses which are counted.

Also this time period can be measured by using an intermittent motor, since each revolution of this intermittent motor consists of a large number of steps, for example 96, which are electronically counted. Also by using a time measuring device the time period can be measured if the speed of the carrier is known. By conversion or reduction it is possible to determine the stretch of displacement of the carrier.

Also a sliding-contact fixed to the carrier and which slides over a carbon rheostat or a rheostat of wounded wire can be used. The resistance is a measure for the displacement of the carrier. Another method makes use of a pick-up, fixed to the carrier, which slides over a magnetic band or strip. Pulses at a fixed distance on the magnetic band are detected by the pick-up and then amplified. Another method makes use of a laser-beam, which by directing it towards a disc or strip containing a regular unevenness, is intermittently reflected to a photo-sensor.

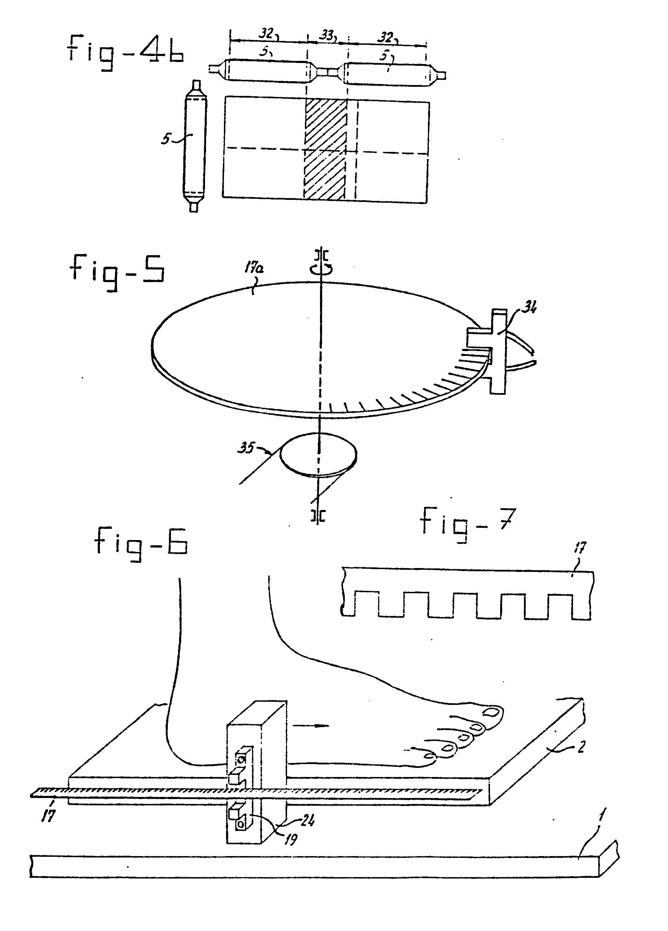

Figure 5 shows an example of the general plan of a slitted disc 17a together with a sensor 34. By rotating the disc the light sensor will be intermittently illuminated and not-illuminated so that pulses are generated and electronically counted. The drive gear 35 of the disc is coupled to the movement of the carrier with the photo-sensors which moves along the foot. With this slitted-disc there is need for a rather long driving chain which will influence the precision of the measurement. The problem is that the displacement of the carriers with the sensing means and the rotation of the slitted disc are not directly coupled, but through several transmissions and the corner pulley.

That is the reason that preferably instead of a slitted disc, a slitted strip is used.

Figure 6 shows this slitted strip fixed onto the foundation-plate 1 beside the foot-receiving platform 2 in the longitudinal direction. At the rear side of the carrier 24 is mounted a photo-sensing system 19 that moves along the slitted strip 17 to determine the length of the longitudinal stretch. So the displacement of the carrier is measured directly and by eliminating the rotating slitted disc 17a there are less moving parts and also the slitted strip 17 needs little room. The length of the slitted strip is about 400 mm., which is the maximum possible length of stretch needed to be measured. In the slitted strip, 0,5 mm large slits are milled at a mutual distance of 0,5 mm. So the total number of slits is about 400.

Figure 7 shows a part of the slitted strip 17. In principle each change from light to darkness and darkness to light will cause a pulse. Thus slits, 0,5 mm large, will generate two pulses each millimeter. That means that the accuracy deviation is less than 1 mm. So the size detected has a mean tolerance of $\pm 0,5$ mm. By using a second sensor which is placed $\pm 0,25$ mm off with regards to the first sensor, the accuracy will be two times better. However the digital reading makes the accuracy a little less again. The same method can be used to measure the width of the foot. The consequences will be that as the lateral dimension is smaller, errors will be of greater influence. Thus the accuracy will be less and another slitted strip of a length of about 200 mm, light sensors and pulse generators have to be used, so that the whole measuring device has to be doubled.

Also, according to the invention, an element according to the reflection- or transparent principle could be used for measuring the displacement, for example by connecting to the carrier a light and reflection head which moves along a straight measuring line. Also a Hall-generator could be used.

Also advantage can be taken of the fact that between the driving-mechanism of the two carriers in the longitudinal and in the lateral direction there is a fixed ratio which, according to the already mentioned dimensions, is 2.5:1. This fixed ratio is obtained by using different diameters for the driving-wheels, which are mounted on the central corner axes, and placed at the beginning of the longitudinal and lateral stretch.

Above is mentioned that for each millimeter in the longitudinal direction four pulses are generated, which amounts to a total of 1500 pulses for a measured stretch of 375 mm. During the time the carrier in the longitudinal direction moves along the stretch of 375 mm, the carrier in the lateral direction moves along a stretch of 150 mm. To measure that stretch of 150 mm, in principle, 1500 pulses are available. At the displacement of 1 mm 10 pulses will be generated, so that in the lateral direction the measurements can be taken with an accuracy of ±0,2 mm. During the simultaneous movement of the carriers for the measurements in the longitudinal direction and in the lateral direction advantageous use can be made of the pulses generated by only one slitted strip with one light source and two radiant energy sensing elements.

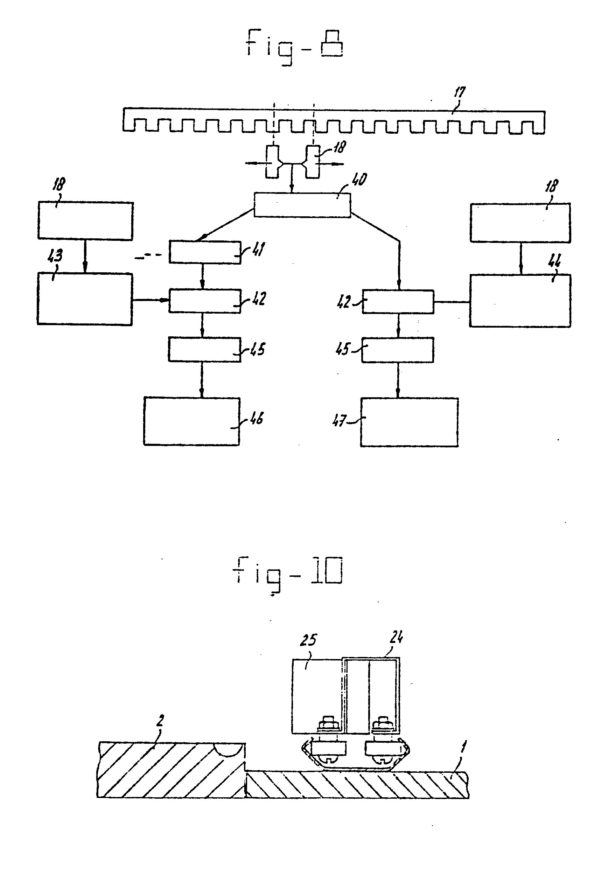

Fig. 8 shows a system diagram of the measuring system. The signals obtained during the movement and caused by the change from darkness to light and from light to darkness, have to be converted to pulses. The total need is four pulse shapers 40, two for each light sensor 18 for the change from light-to-dark and from dark to light has to be measured. For the measurement of the length a binary scaler 41 is needed. The digital reading has an accuracy of half a millimeter, so the first decimal has to be a zero or a five. There are four pulses to each millimeter but first this number is electronically divided by two. Thus there are two signals each millimeter. The obtained reading will be as follows:

| Pulses | Divided | Signal | Reading |
|--------|---------|--------|---------|
| 0 | 0 | no | 0,0 |
| 1 | ($\frac{1}{2}$) | no | 0,0 |
| 2 | 1 | yes | 0,5 |
| 3 | ($1\frac{1}{2}$) | no | 0,5 |
| 4 | 2 | yes | 1,0 |
| 5 | ($2\frac{1}{2}$) | no | 1,0 |

For the measurement of the width there is no need for a divider as the reading is within an accuracy of 0,2 mm and therefore 10 pulses are needed each millimeter.

The initial- and stop signal, respectively 43 and 44, produced by the light sensing elements 18, for the measurements in respectively the longitudinal and lateral direction, determine the time period during which the relevant AND-gate 42 is open and the pulses from the slitted strip-photo sensing device can pass through. These pulses are counted by *counters* 45 and reproduced on the read-out panels 46 and 47 and computed into millimeters.

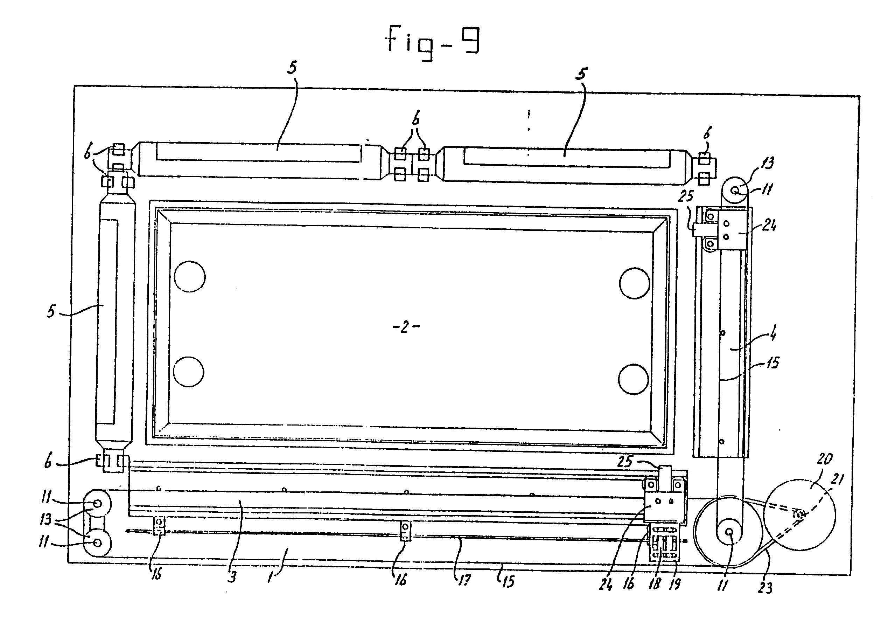

Fig. 9 is a topview of the removable foot-receiving platform 2 surrounded by the foundation-plate 1 on which are mounted the measuring- and driving arrangement. In this figure the carriers are placed on guides, one in the longitudinal direction 3 and one in the lateral direction 4. By means of clamps 6, oblong lamps 5 are fixed in lampholders 7. The driving wheels 14 and guide wheels 13 for the measurements in the longitudinal and lateral direction are mounted on the axis 11 and over the wheels is placed a nyloncord. In the stripholder 16 is mounted the slitted strip 17. The driving-wheel 14 is connected to the motor-wheel 21 of the motor 20 by means of an O-ring 23. At the rearside of the carrier 24 is mounted a photo-detecting arrangement 19 with two sensors 18. The sensors 18 are mounted in the holder 25.

The carriers are placed on guides or rails 3 and 4 which are, for example, made out of 6 cm wide and 1 mm thick sheet steel of which the boards are flanged twice. The carriers 24 run on three ballbearings 26 in this V-groove. In the horizontal plane this system has self-righting qualities.

The central drivewheel system consists of two wheels, above each other, of which the upper wheel has a diameter of 20 mm and this is used for the driving of the carrier for the measurements carried out in the lateral direction and the lower wheel with a diameter of 50 mm is for the driving of the carrier in the longitudinal direction. The ratio between the diameters of the two wheels is thus 2,5.

To prevent that a winding of the cord will cross over itself which would cause a change in the diameter of the turns and in the ratio of transformation, the nylon cord is led through a spiral groove on the periphery or outside surface of each wheel. To avoid any displacement of the carriers with respect to each other when moved, a hole is made in the periphery or outer surface of the wheel, in which the cord is fixed in order to obtain an extra non-skid mechanism. The driving wheels are made out of brass and nickelplated to avoid excess wear and tear of the cord and the three other guide-wheels at the end of each measuring stretch are made out of Estalon, which has excellent skid qualities.

In the holders of the carriers above each other five photo-cells are mounted and in addition two sensors for the counting are mounted on the carrier for the measurement in the longitudinal direction. Both carriers need electric current which is realised by means of a large ribbon, which is flexible in the longitudinal direction and rather stiff in the lateral direction. Instead of a ribbon a thin and flexible foil containing conducting tapes could be used.

By fixing the ribbon or tape half way up the measuring stretch on the foundation-plate and then to connecting it to the carrier following a curved line, the movement of the ribbon or tape is well defined and moves vertically free in a fixed track. The problem of the need of electric current for the carriers can be solved by mounting the photo-cells at a fixed place and by mounting mirrors on the carriers.

As the carriers are coupled to each other by the cord, there is only need for two end switches on one stretch to stop the movement of the carriers at the end of both stretches. The direction of rotation of the driving motor is then automatically inverted by one of the end switches, so the measuring apparatus is automatically ready for the next measuring run. The components of the measuring arrangement can be placed inside the measuring apparatus or in a separate box connected to a wall or placed on a socle. The digital reading during the measuring procedure can be suppressed so that only the final results will be visible in digital numbers.

Several improvements are possible within the scope of the invention such as black painting of the holders containing the photo-cells to avoid that light from the sides would reflect and so influence the measurements; also the use of a slot diaphragm in front of the photo-sensors and the use of polarising filters.

## Claims

1. Apparatus for measuring the width and length of a foot, comprising:

(i) a platform (2) adapted to receive the foot placed thereon, with foot aligning means,
(ii) a frame with a foundation plate (1),
(iii) first and second light sources (5) level with the foot to be measured, in respectively one end wall and one side wall of the frame,
(iv) first and second movable carriers (24) in respectively the other end wall and the other side wall of the frame, supporting respectively first and second light sensing means, and
(v) an electrical circuit and a read-out panel,

in use the first and second carriers (24) being simultaneously moved parallel to respectively the width and length dimensions of the foot from respective positions in which the light from the respective light sources is obscured by the foot to respective second positions in which the light is incident on the respective light sensing means, the resultant signals from the respective light sensing means being used by the electrical circuit to determine respectively the width and length of the foot and to display them on the readout panel, characterized in that:

(vi) the first and second light sensing means (25) are respectively a first and a second vertical bank (25) of respectively first and second light sensors,
(vii) in use the first light sensing means moves from a first position where light is incident on all the first sensors, through a second position aligned with one of the two side extremities of the foot in which the light to at least one of the first sensors is obscured by the foot, to a third position at the other side extremity of the foot in which the light reaches all the first sensors again,
(viii) in use the second light sensing means moves from a fourth position where light is incident on all the second sensors, through a fifth position aligned with one of the two end extremities of the foot in which the light to at least one of the second sensors is obscured by the foot, to a sixth position at the other end extremity of the foot in which the light reaches all the second sensors again, and

the electrical circuit being adapted to produce and process:

(ix) the signals produced at the second and third positions to determine the width of the foot, and
(x) the signals produced at the fifth and sixth positions to determine the length of the foot.

2. Apparatus according to claim 1, characterized in that a light source (5), comprising an oblong light source of the same dimensions as each light sensing means, is mounted on a light source carrier which runs with the same speed as the carrier of the corresponding light sensing means.

3. Apparatus according to one of the previous claims, characterized in that a motor (20) is used for the driving of the carriers (24) and a common coupling device (13, 14) is provided which has such a gear ratio that in the same period of time each carrier is transported along its stretch.

## Revendications

1. Appareil pour la mesure de la largeur et longueur d'un pied, comprenant:

(i) une plate-forme (2) adaptée à recevoir le pied posé dessus, avec des moyens d'alignement du pied,
(ii) un cadre avec une plaque de fondation (i),
(iii) une première et une deuxième source de lumière (5) au nieveau avec le pied à mesurer, respectivement dans une paroi d'extrémité et un paroi latérale du cadre,
(iv) un premier et un second support mobile (24) respectevement dans l'autre paroi d'extrémité et l'autre paroi latérale du cadre, supportant respectivement un premier et un second moyen sensible à la lumière, et
(v) un circuit électrique et un panneau de lecture,

en fonctionnement le premier et le second support mobile (24) etant simultanément déplacés parallêlement et respectivement à la dimension de longueur et largeur du pied, respectivement des positions ou la lumière venant des sources de lumière correspondantes est obscurcie par le pied vers les seconds positions respectivements ou la lumière est incident sur les moyens sensible à la lumière, les signaux resultants des moyens sensible à la lumière respectivements etant utilisés par le circuit électrique pour déterminer respectivement la largeur et la longueur du pied et pour les montrer sur le panneau de lecture, charactérisé en ce que,

(vi) le premier et second moyen sensible à la lumière (25) sont respectivement une première et un seconde batterie (25) du respectivement premier et second sensor de la lumière,

(vii) en fonctionnement le premier moyen sensible à la lumière se déplace d'une première position d'ou la lumière est incidente sur tous les premiers sensors, par une deuxième position alignée à l'une des deux extrémité laterale du pied où la lumière vers au moins un des premier sensor est obscurcie par le pied, à une troisième position à l'autre extrémité laterale du pied d'où la lumière atteint tous les premiers sensors de nouveau,

(viii) en fonctionnement le second moyen sensible à la lumière se déplace d'une quatrième position d'où la lumière est incidente sur tous les seconds sensors, par une cinquième position alignée à l'une de deux extrémité longitudinal du pied d'où la lumière vers au moins un des seconds sensor est obscurcie par le pied, à une sixième position à l'autre extrémité longitudinale du pied d'où la lumière atteint tous les seconds sensors du nouveau, et

le circuit électrique etant adapté de produire et processus:

(ix) les signaux procuits à la seconde et troisième positions pour déterminer la largeur du pied, et

(x) les signaux produits à la cinquième et sixième positions pour déterminer la longueur du pied.

2. Appareil selon la revendication 1, caractérisé en ce que une source de lumière, comprenant une source lumineuse (5) oblong des même dimensions que chaque moyen sensible à la lumière, est montée sur un support mobile qui se·déplaçe à la même vitesse que le support mobile du moyen sensible à la lumière correspondante.

3. Appareil selon l'une des revendication 1 ou 2, caractèrisé en ce que un moteur (20) est utilisé pour actioner les support (24) et une disposition a couplage commun (13, 14) est prévue qui à un tel rapport de transmission, que dans la même periode de temps chaque support est transporté le long de son trajet.

**Patentansprüche**

1. Apparat zur Messung der Breite und Länge eines Fusses, bestehend aus:

(i) einer Standfläche (2) zum Aufsetzen des Fusses mit Vorrichtungen für die Fussausrichtung,

(ii) einem Rahmen mit einer Grundplatte (1),

(iii) einer ersten und einer zweiten Lichtquelle (5), die sich höhengleich mit dem zu messenden Fuss in respektive einen der Stirnwände und einen der Seitenwände des Rahmens befinden,

(iv) einem ersten und einem zweiten beweglichen Mitnehmer (24) in respektive der anderen Stirnwand und der anderen Seitenwand des Rahmens, an denen ersten und zweiten lichtempfindlichen Mitteln befestigd sind, und

(v) einem elketrischen Schaltkreis und einer Anzeigetafel,

wenn in Betrieb der erste und der zweite Mitnehmer (24) gleichzeitig parallel zur respektive Breiten- und Längenrichtung des Fusses aus respektive den Positionen, in der das Licht der lichtquellen vom Fuss verdeckt wird, in die respektive zweite Positionen, in der das Licht in die respektive lichtempfindlichen Mitteln einfällt, bewegt werden und die daraus resultierenden Signale der lichtempfindlichen Mitteln von dem elektrischen Schaltkreis zur Feststellung der Breite und Länge des Fusses und zu ihrer Anzeige auf der Anzeigetafel benutzt werden, dadurch gekennzeichnet, dass:

(vi) die ersten und die zweiten lichtempfindlichen Mitteln (25) respektive eine erste und eine zweite vertikale Batterie (25) von ersten und zweiten Lichtsensoren darstellen,

(vii) wenn in Betrieb die ersten lichtempfindlichen Mitteln aus einer ersten Position, in der das Licht in alle ersten Sensoren einfällt, durch eine zweite Position in Höhe einer der beiden Seitenkanten des Fusses, in der das Licht zu mindestens einem der ersten Sensoren durch den Fuss verdeckt wird, in eine dritte Position an der anderen Seitenkante des Fusses, in der das Licht wieder in alle ersten Sensoren einfällt, bewegt werden,

(viii) wenn in Betrieb die zweiten lichtempfindlichen Mitteln aus einer vierten Position, in der das Licht in alle zweiten Sensoren einfällt, durch eine fünfte Position in Höhe einer der beiden Endkanten des Fusses, in der das Licht zu mindestens einem der zweiten Sensoren durch den Fuss verdeckt wird, in eine sechste Position an der anderen Endkante des Fusses, in der das Licht wieder in alle zweiten Sensoren einfällt, bewegt werden, und

dass der elektrische Schaltkreis eingerichtet ist für die Auslösung und Verarbeitung:

(ix) der in der zweiten und dritten Position ausgelösten Signale zur Bestimmung der ·Breite des Fusses und

(x) der in der Fünften und sechsten Position ausgelösten Signal zur Bestimmung der Länge des Fusses.

2. Apparat nach Anspruch 1, dadurch gekennzeichnet, dass eine Lichtquelle (5), bestehend aus einer rechteckigen Lichtquelle derselben Abmessungen wie jeder Lichtempfindliche Mitteln, an einem Lichtquellenmitnehmer befestigt ist, der sich mit derselben Geschwindigkeit bewegt wie die korrespondierende lichtempfindliche Mitteln.

3. Apparat nach einem der vorstehenden Ansprüche, dadurch gekennzeichnet, dass ein Motor (20) für den Antrieb der Mitnehmer (24) verwendet wird und eine gemeinsame Kupplungsvorrichtung (13, 14) vorgesehen ist, deren Übersetzungsverhältnis so beschaffen ist, dass jede Mitnehmer ihr Bahnstrecke in der gleichen Zeitspanne zurücklegen.

# Fig-1

# Fig-2a

# Fig-2b

fig-2c

fig-3

fig-4a

**fig-4b**

32  33  32

5  5

5

**fig-5**

17a

34

35

**fig-6**

**fig-7**

17

17

24

19

2

1

## Fig-9

## Fig-10

Fig-9